# EUROPEAN PATENT APPLICATION

(11) **EP 4 375 369 A1**
(43) Date of publication of application: **29.05.2024**
(21) Application number: 22861500.1
(22) Date of filing: 30.03.2022
(51) Int. Cl.: C12N 9/10, C12N 15/52, C12P 13/08, C12N 15/77

(54) **NOVEL ACETOHYDROXY ACID SYNTHASE SUBUNIT VARIANT AND METHOD FOR PRODUCING L-VALINE USING SAME**

(30) Priority: 23.08.2021 KR 20210110890
(71) Applicant: CJ CheilJedang Corporation, Seoul 04560 (KR)
(72) Inventor: OH, Haena, Seoul 04560 (KR); KIM, Seon Hye, Seoul 04560 (KR); LEE, Heeseok, Seoul 04560 (KR); YUN, Hyojin, Seoul 04560 (KR); YOON, Byoung Hoon, Seoul 04560 (KR); KIM, Ju-yeon, Seoul 04560 (KR); CHOI, Sun Hyoung, Seoul 04560 (KR); SHIM, Jihyun, Seoul 04560 (KR)
(74) Representative: Weickmann & Weickmann PartmbB
(86) International application number: PCT/KR2022/004474
(87) International publication number: WO 2023/027284

(57) **Abstract**

The present application relates to: a novel acetohydroxy acid synthase subunit (ilvN) variant; a polynucleotide encoding the variant; an expression vector comprising the polynucleotide; microorganisms producing L-valine including the acetohydroxy acid synthase subunit (ilvN) variant; and a method for producing L-valine using the microorganisms.

## Description

### [Technical Field]

The present application relates to a novel acetohydroxy acid synthase small subunit (ilvN) variant, a polynucleotide encoding the variant, an expression vector including the polynucleotide, an L-valine producing microorganism including the acetohydroxy acid synthase small subunit (ilvN) variant, and a method for producing L-valine using the microorganism.

### [Background Art]

L-Amino acids are basic structural units of proteins and used as important materials for pharmaceuticals, food additives, animal feeds, nutrients, pesticides, bactericides, and the like. In particular, branched-chain amino acid (BCAA) is a generic term for L-valine, L-leucine, and L-isoleucine, which are essential amino acids, and the branched-chain amino acids are known to have an antioxidant effect and an effect of directly promoting protein synthesis in muscle cells.

Meanwhile, the branched-chain amino acids are mainly produced by microorganisms of the genus *Escherichia* or *Corynebacterium* and are known to be biosynthesized from 2-ketoisocaproate as a precursor after undergoing several steps from pyruvic acid (Korean Patent Publication Nos. 10-0220018 and 10-0438146). However, the production of L-branched-chain amino acids by the microorganisms poses challenges in achieving large-scale industrial production.

Under these circumstances, the present inventors verified that the introduction of a variant with enhanced activity of the gene *ilvN* (acetohydroxy acid synthase small subunit) encoding an enzyme involved in the microbial L-valine biosynthesis, for the purpose of improving L-valine producing ability by using microorganisms, significantly increased L-valine producing ability.

### [Disclosure]

### [Technical Problem]

The present inventors developed a novel acetohydroxy acid synthase small subunit variant increasing L-valine production, a polynucleotide encoding the variant, an expression vector including the polynucleotide, an L-valine producing microorganism including the acetohydroxy acid synthase small subunit variant, and a method for producing L-valine using the microorganism, and thus completed the present application.

### [Technical Solution]

In an accordance with an aspect of the present application, there is provided an acetohydroxy acid synthase small subunit (ilvN) variant in which the amino acid corresponding to position 44 in the amino acid sequence of SEQ ID NO: 1 is substituted with another amino acid.

In another accordance with an aspect of the present application, there are provided a polynucleotide encoding the variant of the present application and an expression vector containing the polynucleotide.

In still another accordance with an aspect of the present application, there is provided a microorganism including the variant of the present application or a polynucleotide encoding the variant.

In still another accordance with an aspect of the present application, there is provided a method for producing L-valine, the method including culturing the microorganism in a medium.

In still another accordance with an aspect of the present application, there is provided a composition for producing L-valine, the composition containing a microorganism including the variant of the present application, a polynucleotide encoding the variant, a vector containing the polynucleotide, or the polynucleotide of the present application; a medium obtained by culturing the microorganism; or a combination of two or more thereof.

In still another accordance with an aspect of the present application, there is provided use of an acetohydroxy acid synthase small subunit variant for producing L-valine, the variant having a substitution of the amino acid at position 44 in the amino acid sequence of SEQ ID NO: 1 with another amino acid.

### [Advantageous Effects]

The culture of microorganisms including the acetohydroxy acid synthase small subunit variant of the present application enables the production of L-valine with high yield compared with microorganisms having an existing unmodified polypeptide.

### [Best Mode for Carrying Out the Invention]

The present application will be specifically described as follows. Each description and embodiment disclosed in the present application may also be applied to other descriptions and embodiments. That is, all combinations of various elements disclosed in the present application fall within the scope of the present application. Furthermore, the scope of the present application is not limited by the specific description below. Furthermore, throughout the overall specification, many papers and patent documents are referenced and their citations are provided. The disclosures of cited papers and patent documents are entirely incorporated by reference into the present specification, and the level of the technical field within which the present invention falls and details of the present invention are explained more clearly.

In accordance with an aspect of the present application, there is provided an acetohydroxy acid synthase small subunit (ilvN) variant in which the amino acid corresponding to position 44 in the amino acid sequence of SEQ ID NO: 1 is substituted with another amino acid.

As used herein, the term "L-valine" refers to an L-amino acid, corresponding to one of the essential amino acid, which has a chemical formula of (CH₃)₂CHCH(NH₂)COOH, structurally belonging to branched-chain amino acids, together with L-leucine and L-isoleucine.

As used herein, the term "acetohydroxy acid synthase" refers to the first enzyme in L-valine biosynthesis and is also called acetolactate synthase. Acetohydroxy acid synthase can catalyze the decarboxylation of pyruvate and a condensation reaction with an another pyruvate molecule to produce acetolactate, which is a precursor of valine, or catalyze the decarboxylation of pyruvate and a condensation reaction with 2-ketobutyrate to produce acetohydroxybutyrate, which is a precursor of isoleucine.

Acetohydroxy acid synthase is encoded by the two genes *ilvB* and *ilvN*, wherein the *ilvB* gene encodes an acetohydroxy acid synthase large subunit and the *ilvN* gene encodes an acetohydroxy acid synthase small subunit. Of these, the small subunit encoded by the *ilvN* gene is considered to be significantly involved in the feedback inhibition.

The acetohydroxy acid synthase encoded by the *ilvN* gene may have the amino acid sequence of SEQ ID NO: 1, but is not particularly limited thereto.

The variant of the present application may have enhanced activity by the substitution of an amino acid at a specific position in the existing amino acid sequence of the acetohydroxy acid synthase small subunit (ilvN), but is not limited thereto.

In an embodiment, the acetohydroxy acid synthase small subunit (ilvN) variant may be an acetohydroxy acid synthase small subunit (ilvN) variant including at least one amino acid substitution in the amino acid sequence of SEQ ID NO: 1, but is not limited thereto.

Specifically, in the variant of the present application, the amino acids corresponding to position 44 and/or 42 may be substituted with other amino acids, but are not limited thereto. More specifically, in the variant, the amino acids at any one or both of the above-cited positions or at positions corresponding thereto may be substituted with other amino acids, but is not limited thereto.

The "another amino acid" or "other amino acids" is not limited as long as the amino acids differ from the amino acids before the substitutions. For example, the wording "the amino acid corresponding to position 44 in SEQ ID NO: 1 is substituted with another amino acid" may mean that the amino acid is substituted with alanine, phenylalanine, glycine, arginine, aspartate, cysteine, glutamate, asparagine, glutamine, histidine, proline, serine, tyrosine, isoleucine, lysine, tryptophan, valine, methionine, or leucine, other than threonine, and the wording "the amino acid corresponding to position 42 in SEQ ID NO: 1 is substituted with another amino acid" means that the amino acid is substituted with valine, asparagine, glycine, arginine, aspartate, cysteine, glutamic acid, glutamine, histidine, proline, serine, tyrosine, isoleucine, leucine, lysine, phenylalanine, tryptophan, methionine, or threonine, other than alanine, but is not limited thereto.

Meanwhile, a person skilled in the art can understand amino acids corresponding to positions 44 and 42 in SEQ ID NO: 1 of the present application in any amino acid sequence through sequence alignment known in the art, and even if not separately described herein, it would be obvious that the wording "amino acid at a specific position in a specific sequence number" means the inclusion of even "an amino acid at a position corresponding" thereto in any amino acid sequence. Therefore, an amino acid sequence, in which at least one amino acid selected from the group consisting of the amino acids corresponding to positions 44 and 42 in SEQ ID NO: 1 is substituted with another amino acid, is also included in the range of the present application.

For example, the substitution of at least one amino acid of the amino acids corresponding to positions 44 and 42 in SEQ ID NO: 1 with another amino acid can provide a variant having higher activity than the non-substituted (non-modified) amino acid sequence.

Specifically, in the variant of the present application, the amino acids corresponding to position 44 and/or position 42 may be substituted with other amino acids, but are not limited thereto.

As a specific example, in the variant of the present application, threonine, the amino acid corresponding to position 44 in SEQ ID NO: 1 may be substituted with alanine and/or alanine, the amino acid corresponding to position 42 in SEQ ID NO: 1 may be substituted with valine, but are not limited thereto.

As a more specific example, the variant of the present application may have the amino acid sequence set forth in SEQ ID NO: 3 or 5 or may essentially consist of the amino acid sequence.

In addition, the variant of the present application may include not only a variant having the amino acid sequence set forth in SEQ ID NO: 3 or 5 but also a variant having at least 80%, specifically at least 90%, more specifically at least 95%, and still more specifically at least 99% homology to the amino acid sequence, wherein the amino acids at positions corresponding to the amino acid at position 44 and/or the amino acid at position 42 from the N-terminus of SEQ ID NO: 1 may be substituted with other amino acids. It would be obvious that any variant that has an amino acid sequence with a deletion, modification, substitution, conservative substitution, or addition in a part thereof may also be included within the range of the present application as long as the amino acid sequence has such homology or identity and exhibits efficacy corresponding to the variant of the present application.

Examples thereof may include variants having a sequence addition or deletion, a naturally occurring mutation, a silent mutation, or a conservative substitution that does not alter functions of the variant of the present application at the N-terminus, C-terminus, and/or inside of the amino acid sequence.

As used herein, the term "conservative substitution" refers to a substitution of one amino acid with another amino acid having similar structural and/or chemical properties. Such an amino acid substitution may be generally made on the basis of similarity in polarity, charge, solubility, hydrophobicity, hydrophilicity, and/or amphipathic nature of the residues. For example, positively charged (basic) amino acids include arginine, lysine, and histidine; negatively charged (acidic) amino acids include glutamic acid and aspartic acid; aromatic amino acids include phenylalanine, tryptophan, and tyrosine; and hydrophobic amino acids include alanine, valine, isoleucine, leucine, methionine, phenylalanine, tyrosine, and tryptophan. In addition, amino acids may be classified into amino acids with electrically charged side chains and amino acids with electrically uncharged side chains. The amino acids with electrically charged side chains include aspartic acid, glutamic acid, lysine, arginine, and histidine, and the amino acids with electrically uncharged side chains may be further classified into nonpolar amino acids or polar amino acids. The nonpolar amino acids may include glycine, alanine, valine, leucine, isoleucine, methionine, phenylalanine, tryptophan, and proline, and the polar amino acids include serine, threonine, cysteine, tyrosine, asparagine, and glutamine. Typically, conservative substitutions have little or no effect on the activity of a produced polypeptide. Typically, the conservative substitutions may have little or no effect on the activity of a protein or polypeptide.

In addition, the variant may include deletions or additions of amino acids that have a minimal effect on the properties and secondary structures of a polypeptide. For example, the polypeptide may be conjugated to a signal (or leader) sequence of the N-terminal of a protein involved in the transfer of the protein co-translationally or post-translationally. The polypeptide may also be conjugated to another sequence or linker to identify, purify, or synthesize the polypeptide.

As used herein, the term "variant" refers to a polypeptide which has a different sequence but maintain functions or properties compared with the amino acid sequence before mutation by a conservative substitution and/or a modification of at least one amino acid. Such a variant can be generally identified by modifying at least one amino acid in the amino acid sequence of the polypeptide and evaluating properties of the modified polypeptide. That is, the ability of the variant may be increased, unchanged, or reduced compared with the polypeptide before mutation. Some variants may include a variant in which at least one part, such as an N-terminal leader sequence or a transmembrane domain, is removed. Other variants may include a variant in which a part of the N-terminus and/or C-terminus of a mature protein is removed. The term "variant" may also be used interchangeably with "modification", "modified polypeptide", "modified protein", "mutant", "mutein", "divergent", or the like, and any term that is used in a sense of being mutated can be used without limitation thereto. For the purpose of the present application, the variant may be a polypeptide including: the amino acid sequence set forth in SEQ ID NO: 3 in which threonine, the amino acid corresponding to position 44 in SEQ ID NO: 1 is substituted with alanine; or the amino acid sequence set forth in SEQ ID NO: 5 in which alanine, the amino acid corresponding to position 42 in SEQ ID NO: 1 is substituted with valine.

As used herein, the term "homology" or "identity" refers to the degree of similarity between two given amino acid sequences or nucleotide sequences and may be expressed as a percentage. The terms homology and identity may be often used interchangeably with each other.

The sequence homology or identity of conserved polynucleotides or polypeptides may be determined by standard alignment algorithms, and default gap penalties established by a program to be used may be used together. Substantially, homologous or identical sequences may generally hybridize with each other as a whole or in part under moderate or highly stringent conditions. It would be obvious that the hybridization also includes hybridization with a polynucleotide containing usual codons or codons considering codon degeneracy in the polynucleotide.

The homology, similarity, or identity between any two polynucleotide or polypeptide sequences may be determined using a known computer algorithm, such as the "FASTA" program, by using default parameters as in Pearson et al., (1988) Proc. Natl. Acad. Sci. USA 85:2444. Alternatively, this may be determined using the Needleman-Wunsch algorithm (Needleman and Wunsch, 1970, J. Mol. Biol. 48:443-453), which is performed in the Needleman program of the European Molecular Biology Open Software Suite (EMBOSS) package (Rice et al., 2000, Trends Genet. 16:276-277) (version 5.0.0 or later) (including GCG program package (Devereux, J., et al, Nucleic Acids Research 12:387 (1984)), BLASTP, BLASTN, FASTA (Atschul, S. F., et al., J MOLEC BIOL 215:403 (1990); Guide to Huge Computers, Martin J. Bishop, ed., Academic Press, San Diego,1994, and CARILLO et al. (1988) SIAM J Applied Math 48:1073). For example, the homology, similarity, or identity may be determined using BLAST of the National Center for Biotechnology Information database, or ClustalW.

The homology, similarity, or identity between the polynucleotides or polypeptides may be determined by comparing sequence information using the GAP computer program, for example, Needleman et al., (1970), J Mol Biol. 48:443, as known in Smith and Waterman, Adv. Appl. Math (1981) 2:482. Briefly, the GAP program defines similarity as the number of aligned symbols (i.e., nucleotides or amino acids) which are similar, divided by the total number of symbols in the shorter of the two sequences. The default parameters for the GAP program may include: (1) a binary comparison matrix (containing a value of 1 for identity and 0 for non-identity) and the weighted comparison matrix of Gribskov et al. (1986) Nucl. Acids Res. 14:6745 as disclosed in Schwartz and Dayhoff, eds., Atlas of Protein Sequence And Structure, National Biomedical Research Foundation, pp. 353-358 (1979) (or the EDNAFULL (EMBOSS version of NCBI NUC4.4) substitution matrix); (2) a penalty of 3.0 for each gap and an additional 0.10 penalty for each symbol in each gap (or a gap open penalty of 10, and a gap extension penalty of 0.5); and (3) no penalty for end gaps.

In an embodiment of the present application, the variant of the present application may be obtained by a mutation of an L-valine producing microorganism. The mutation of the microorganism may be performed by various means known in the art and may use one of physical or chemical mutagenic factors. Examples of the chemical mutagenic factor suitable for the present invention include *N*-methyl-*N'*-nitro-*N*-nitrosoguanidine (NTG), diepoxybutane, ethyl methane sulfonate, mustard compounds, hydrazine, and nitrous acid, but are not limited thereto. Examples of the physical mutagenic factor may include ultraviolet and gamma radiation, but are not limited thereto.

In the present application, the expression of the acetohydroxy acid synthase small subunit (ilvN) may be enhanced, and the enhancement of the expression can increase L-valine producing ability.

As used herein, the term "corresponding to" refers to amino acid residues at positions listed in the polypeptide or amino acid residues that are similar, identical, or homologous to those listed in the polypeptide. Identifying the amino acid at the corresponding position may be determining a specific amino acid in a sequence that refers to a specific sequence. As used herein, the term "corresponding region" generally refers to a similar or corresponding position in a related protein or reference protein.

For example, any amino acid sequence is aligned with SEQ ID NO: 1, and based on this, each amino acid residue of the amino acid sequence may be numbered with reference to an amino acid residue numerical position corresponding to an amino acid residue of SEQ ID NO: 1. For example, a sequence alignment algorithm as described in the present application may identify the position of an amino acid or a position of occurrence of a modification, such as a substitution, insertion, or deletion, compared with a query sequence (also referred to as a "reference sequence").

For example, Needleman-Wunsch algorithm (Needleman and Wunsch, 1970, J. Mol. Biol. 48:443-453) or Needle program of the EMBOSS package (EMBOSS: The European Molecular Biology Open Software Suite, Rice et al., 2000, Trends Genet. 16:276-277) may be used for such alignment, but without limitation thereto, a sequence alignment program, a pairwise sequence comparison algorithm, or the like, which are known in the art, may be used as appropriate.

In accordance with another aspect of the present application, there is provided a polynucleotide encoding the variant of the present application.

As used herein, the term "polynucleotide" refers to a polymer of nucleotides chain-extended lengthwise by a covalent bond of nucleotide monomers, and in general a DNA or RNA strand with a certain length, and more specifically, may mean a polynucleotide fragment encoding the variant.

The polynucleotide encoding the variant of the present application may include a nucleotide sequence encoding the amino acid sequence set forth in SEQ ID NO: 3 or 5. As an example, the polynucleotide of the present application may have or include the sequence of SEQ ID NO: 4 or 6. Alternatively, the polynucleotide of the present application may consist of or essentially consist of the sequence of SEQ ID NO: 4 or 6.

In the polynucleotide of the present application, various modifications may be made in the coding region as long as the amino acid sequence of the variant of the present application is not changed in consideration of codon degeneracy or codons preferred in an organism that is intended to express the variant of the present application. Specifically, the polynucleotide of the present application may have or include a nucleotide sequence having at least 70%, at least 75%, at least 80%, at least 85%, at least 90%, at least 95%, at least 96%, at least 97%, or at least 98%, but less than 100% homology or identity to the sequence of SEQ ID NO: 4 or 6, or may consist of or consist essentially of a nucleotide sequence having at least 70%, at least 75%, at least 80%, at least 85%, at least 90%, at least 95%, at least 96%, at least 97%, or at least 98%, but less than 100% homology or identity to the sequence of SEQ ID NO: 4 or 6, but is not limited thereto. Particularly, in the sequences having homology or identity, the codon encoding the amino acid corresponding to position 44 in SEQ ID NO: 3 or 5 may be one of the codons encoding alanine, and the codon encoding the amino acid corresponding to position 42 in SEQ ID NO: 5 may be one of the codons encoding valine.

In addition, the polynucleotide of the present application may include, without limitation, a sequence that can hydride, under stringent conditions, with a probe capable of being prepared from a known gene sequence, for example, a sequence complementary to a part of or the entire polynucleotide sequence of the present application. The term "stringent condition" refers to a condition that enable specific hybridization between polynucleotides. These conditions are specifically described in the literature (see J. Sambrook et al., Molecular Cloning, A Laboratory Manual, 2nd Edition, Cold Spring Harbor Laboratory press, Cold Spring Harbor, New York, 1989; and F. M. Ausubel et al., Current Protocols in Molecular Biology, John Wiley & Sons, Inc., New York, 9.50-9.51, 11.7-11.8). For example, the stringent conditions may include a condition in which polynucleotides having higher homology or identity, for example, polynucleotides having at least 70%, at least 75%, at least 80%, at least 85%, at least 90%, at least 95%, at least 96%, at least 97%, at least 98%, or at least 99% homology or identity hybridize with each other but polynucleotides having lower homology or identity do not hybridize with each other; or a condition in which washing is performed one time, specifically two or three times, at a salt concentration and temperature equivalent to 60°C, 1×SSC, 0.1% SDS, specifically 60°C, 0.1×SSC, 0.1% SDS; and more specifically 68°C, 0.1×SSC, 0.1% SDS, which are conditions for common southern hybridization.

Hybridization requires that two nucleic acids have complementary sequences, although depending on the stringency of the hybridization, mismatches between bases are possible. The term "complementary" is used to describe the relationship between nucleotide bases that are capable of hybridizing to one another. For example, with respect to DNA, adenine is complementary to thymine and cytosine is complementary to guanine. Accordingly, the polynucleotide of the present application also includes isolated nucleic acid fragments that are complementary to the complete sequence as well as substantially similar nucleic acid sequences.

Specifically, polynucleotides having homology or identity to the polynucleotide of the present application may be detected by employing hybridization conditions including a hybridization step at Tₘ of 55°C and utilizing the above-described conditions. In addition, the Tₘ value may be 60°C, 63°C, or 65°C, but is not limited thereto, and may be appropriately adjusted by a person skilled in the art according to the purpose.

The appropriate degree of stringency for hybridizing polynucleotides depends on the length of the polynucleotides and the degree of complementarity thereof, and variables thereof are well known in the art (e.g., Sambrook *et al.*, *supra*).

In accordance with still another aspect of the present application, there is provided a vector including the polynucleotide of the present application. The vector may be an expression vector for expressing the polynucleotide in a microorganism, but is not limited thereto.

As used herein, the term "vector" refers to a DNA construct that includes a nucleotide sequence of a polynucleotide encoding a target protein and operably linked to a suitable control sequence so that the target protein can be expressed in an appropriate host. The expression control region may include a promoter capable of initiating transcription, any operator sequence for controlling such transcription, a sequence for encoding a suitable mRNA ribosomal binding site, and a sequence for controlling the termination of transcription and translation. The vector, after transformation into a suitable microorganism, can replicate or function irrespective of the genome of the host or may be integrated into the genome itself.

The vector used in the present application is not particularly limited, and any vector known in the art may be used. Examples of the vector that is commonly used may include natural or recombinant plasmids, cosmids, viruses, and bacteriophages. For example, pWE15, M13, MBL3, MBL4, IXII, ASHII, APII, t10, t11, Charon4A, and Charon21A may be used as phage vectors or cosmid vectors, and pDZ-based, pBR-based, pUC-based, pBluescriptll-based, pGEM-based, pTZ-based, pCL-based, and pET-based vectors may be used as plasmid vectors. Specifically, pDZ, pDC, pDCM2, pACYC177, pACYC184, pCL, pECCG117, pUC19, pBR322, pMW118, and pCC1BAC vectors may be used.

For example, a polynucleotide encoding a target polypeptide may be inserted in a chromosome through a vector for chromosomal insertion in a cell. The insertion of the polynucleotide into the chromosome may be performed using any method known in the art, for example, homologous recombination, but is not limited thereto. The vector may further include a selection maker for investigating the insertion or non-insertion of the chromosome. The selection marker is for selecting cells transformed with the vector, that is, identifying the insertion or non-insertion of a target nucleic acid molecule, and markers imparting a selectable phenotype, such as drug resistance, auxotrophy, cytotoxic drug resistance, or surface polypeptide expression may be used. Under the circumstances of the treatment with a selective agent, only cells expressing selection markers can survive or exhibit other phenotypic traits, so the transformed cells can be selected.

As used herein, the term "transformation" refers to the introduction of a vector containing a polynucleotide encoding a target polypeptide into a microorganism so that the polypeptide encoded by the polynucleotide can be expressed in the microorganism. Examples of the transformed polynucleotide may include any polypeptide as long as it can be expressed in a microorganism, regardless of whether it is inserted and located into the chromosome of the microorganism or located outside of the chromosome of the microorganism. In addition, the polynucleotide includes DNA and/or RNA encoding the target polypeptide. The polynucleotide may be introduced in any form as long as it can be introduced into the microorganism and expressed therein. For example, the polynucleotide may be introduced, into the microorganism, in the form of an expression cassette, which is a gene construct including all the elements required for its self-expression. The expression cassette may generally include a promoter operably linked to the polynucleotide, a transcription termination signal, a ribosome binding site, and a translation terminal signal. The expression cassette may be in the form of a self-replicable expression vector. In addition, the polynucleotide may be introduced in the form as it is into a microorganism and operably linked to a sequence required for expression in the microorganism, but is not limited thereto.

The term "operably linked" as above refers to a functional linkage between the polypeptide sequence and a promoter sequence for initiating and mediating the transcription of a polynucleotide encoding the target variant of the present application.

In accordance with still another aspect of the present application, there is provided a microorganism including the variant of the present application or the polynucleotide of the present application.

The microorganism of the present application may include the modified polypeptide of the present application, a polynucleotide encoding the polypeptide, or a vector containing the polynucleotide of the present application.

As used herein, the term "microorganism (or strain)" encompasses all of wild-type microorganisms or naturally or artificially genetically modified microorganisms, and it may be a microorganism in which a particular mechanism is weakened or enhanced due to the insertion of an exogenous gene or the enhancement or inactivation of activity of an endogenous gene, and may be a microorganism including a genetic modification to produce a desired polypeptide, protein or product. In the present application, the terms "microorganism" and "strain" have the same meaning and can be used interchangeably without limitation.

Specifically, the microorganism may be a microorganism of the genus *Corynebacterium* and, more specifically, *Corynebacterium glutamicum*, but is not limited thereto.

The strain of the present application may be: a strain including at least any one of the variant of the present application, the polynucleotide of the present application, and the vector containing the polynucleotide of the present application; a strain modified to express the variant of the present application or the polynucleotide of the present application; a strain (*e.g*., recombinant strain) expressing the variant of the present application or the polynucleotide of the present application; or a strain (*e.g*., recombinant strain) having activity of the variant of the present application, but is not limited thereto.

The strain of the present application may be an L-valine producing microorganism.

As used herein, the term "L-valine producing microorganism" refers to a prokaryotic or eukaryotic microorganism strain capable of producing L-valine in an organism. For the purpose of the present invention, the microorganism may be a prokaryotic or eukaryotic cell as long as it can produce L-valine by including the acetohydroxy acid synthase small subunit, and examples thereof may include a microorganism strain pertaining to the genus of *Corynebacterium*, for example, *Corynebacterium glutamicum.*

The L-valine producing microorganism including the acetohydroxy acid synthase variant may include both a microorganism, which includes the sequence of the acetohydroxy acid synthase small subunit variant due to the mutation of a gene encoding the acetohydroxy acid synthase on the chromosome, and/or a microorganism, which includes the acetohydroxy acid synthase small subunit variant due to the introduction of a vector containing a polynucleotide encoding the acetohydroxy acid synthase small subunit variant, but is not limited thereto.

Additionally, the L-valine producing microorganism including the acetohydroxy acid synthase small subunit variant may be a microorganism in which the activity of the acetohydroxy acid synthase small subunit variant is enhanced compared with a parent strain.

For example, the strain of the present application is a cell or microorganism that is transformed with a vector including the polynucleotide of the present application or a polynucleotide encoding the variant of the present application to express the variant of the present application, and for the purpose of the present application, the strain of the present application may encompass all the microorganisms capable of producing L-valine by including the variant of the present application. For example, the strain of the present application may be a recombinant strain having enhanced L-valine producing ability through the expression of the acetohydroxy acid synthase small subunit (ilvN) variant by introduction of a polynucleotide encoding the variant of the present application into a native wild-type microorganism or an L-valine producing microorganism. The recombinant strain with enhanced L-valine producing ability may be a microorganism with enhanced L-valine producing ability compared with a natural wild-type microorganism or an acetohydroxy acid synthase non-modified microorganism (*i.e.*, a microorganism expressing wild-type acetohydroxy acid synthase (SEQ ID NO: 1) or a microorganism not expressing a mutant protein (SEQ ID NO: 3 or 5)), but is not limited thereto.

For example, the recombinant strain with enhanced ability may have an L-valine producing ability, which is enhanced by at least about 1%, specifically, at least about 1%, at least about 2.5%, at least about 5%, at least about 6%, at least about 7%, at least about 8%, at least about 9%, at least about 10%, at least about 10.5%, at least about 11%, at least about 11.5%, at least about 12%, at least about 12.5%, at least about 13%, at least about 13.5%, at least about 14%, at least about 14.5%, at least about 15%, at least about 15.5%, at least about 16%, at least about 16.5%, at least about 17%, at least about 17.5%, at least about 18%, at least about 18.1%, at least about 18.2%, at least about 18.3%, at least about 18.4%, at least about 18.5% (the upper bound is not particularly limited, and the upper bound may be, for example, at most about 200%, at most about 150%, at most about 100%, at most about 50%, at most about 45%, at most about 40%, at most about 35%, at most about 30%, at most about 25%, at most about 20%, or at most about 15%), compared with that of the parent strain before modification or the non-modified microorganism, but the L-valine producing ability is not limited thereto as long as it has an increment of a plus (+) value compared with the producing ability of the parent strain before modification or the non-modified microorganism. As another example, the recombinant strain with enhanced producing ability may have an L-valine producing ability, which is enhanced by at least about 1.1 times, at least about 1.12 times, at least about 1.13 times, at least about 1.14 times, at least about 1.15 times, at least about 1.16 times, at least about 1.17 times, or at least about 1.18 times (the upper bound is not particularly limited, and the upper bound may be, for example, at most about 10 times, at most about 5 times, at most about 3 times, at most about 2 times, at most about 1.5 times, or at most about 1.2 times), compared with that of the parent strain before modification or the non-modified microorganism, but the L-valine producing ability is not limited thereto. As used herein, the term "about" refers to a range including ±0.5, ±0.4, ±0.3, ±0.2, ±0.1, and the like, and thus includes all of the values in the range equivalent or similar to those stated after this term, but is not limited thereto.

As used herein, the term "non-modified microorganism" may refers to a wild-type strain or a native strain as it is, or a strain before transformation due to genetic mutation caused by natural or artificial factors, not excluding strains including mutations that may naturally occur in microorganisms. For example, the non-modified microorganism may refer to a strain to which the acetohydroxy acid synthase small subunit (ilvN) variant described herein is not introduced or a strain before the introduction thereof. The "non-modified microorganism" may be used interchangeably with "strain before modification", "microorganism before modification", "non-mutated strain", "non-modified strain", "non-mutated microorganism", or "reference microorganism".

In another embodiment of the present application, the microorganism of the present application may be *Corynebacterium glutamicum*, *Corynebacterium crudilactis, Corynebacterium deserti, Corynebacterium efficiens, Corynebacterium callunae, Corynebacterium stationis, Corynebacterium singulare, Corynebacterium halotolerans, Corynebacterium striatum, Corynebacterium ammoniagenes, Corynebacterium pollutisoli, Corynebacterium imitans, Corynebacterium testudinoris,* or *Corynebacterium flavescens.*

As used herein, the term "enhancement" of polypeptide activity refers to an increase of the activity of a polypeptide compared with intrinsic activity thereof. The enhancement may be used interchangeably with terms, such as activation, up-regulation, overexpression, and increase. Particularly, the activation, enhancement, up-regulation, overexpression, and increase may include exhibiting the activity originally possessed or exhibiting the activity that was improved compared with the intrinsic activity or activity before modification. The term "intrinsic activity" refers to the activity of a specific polypeptide originally possessed by a parental strain before transformation or a non-modified microorganism when the microorganism is transformed by genetic mutation caused by natural or artificially factors. This term may be used interchangeably with the "activity before modification". The "enhancement", "up-regulation", "overexpression", or "increase" of the activity of a polypeptide compared with the intrinsic activity thereof means that the activity and/or concentration (expression level) of a specific polypeptide originally possessed by a parent strain before transformation or a non-modified microorganism is improved.

The enhancement may be achieved through the introduction of an exogenous polypeptide or the increase of the intrinsic activity and/or concentration (expression level) of the polypeptide. The enhancement of the activity of the polypeptide may be identified by an increase in the degree of activity or the expression level of the corresponding polypeptide or an increase in the amount of a product released from the corresponding polypeptide.

For the enhancement of the activity of the polypeptide, various methods well known in the art may be applied, and the method is not limited as long as it can enhance the activity of a target polypeptide compared with that of the microorganism before modification. Specifically, genetic engineering and/or protein engineering well known to those skilled in the art, which are routine methods of molecular biology, may be used, but the method is not limited thereto (*e.g*., Sitnicka et al., Functional Analysis of Genes. Advances in Cell Biology. 2010, Vol. 2. 1-16; and Sambrook et al., Molecular Cloning 2012).

Specifically, the enhancement of the activity of the polypeptide of the present application may be achieved by:
1) increasing the intracellular copy number of a polynucleotide encoding the polypeptide;
2) replacing the expression control region of a gene encoding the polypeptide on the chromosome with a sequence with stronger activity;
3) modifying the nucleotide sequence encoding the initiation codon or 5'-UTR of the gene transcript encoding the polypeptide;
4) modifying an amino acid sequence of the polypeptide such that the activity of the polypeptide is enhanced;
5) modifying a polynucleotide sequence encoding the polypeptide such that the activity of the polypeptide is enhanced (*e.g*., modifying a polynucleotide sequence of the polypeptide gene so as to encode a polypeptide that has been modified to enhance the activity of the polypeptide);
6) introducing an exogenous polypeptide exhibiting the activity of the polypeptide or an exogenous polynucleotide encoding the same;
7) codon optimization of a polynucleotide encoding the polypeptide;
8) modifying or chemical modifying an exposed site selected by analysis of a tertiary structure of the polypeptide; or
9) a combination of two or more selected from 1) to 8) above, but is not particularly limited thereto.

A description is made more specifically as follows.

The increasing of the intracellular copy number of a polynucleotide encoding the polypeptide in item 1) above may be achieved by introducing, into a microorganism, a vector operably linked to the polynucleotide encoding the corresponding polypeptide and able to replicate and function regardless of a host. Alternatively, the increasing may be achieved by introducing one or more copies or more of polynucleotides encoding the corresponding polypeptide into the chromosome of the microorganism. The introduction into the chromosome may be performed by introducing a vector, which is able to insert the polynucleotide into the chromosome of the microorganism, into the microorganism, but is not limited thereto. The vector is as described above.

The replacing of the expression control region (or expression control sequence) of a gene encoding the polypeptide on the chromosome with a sequence with stronger activity in item 2) above may be, for example, inducing a modification on the sequence through deletion, insertion, non-conservative or conservative substitution, or a combination thereof to further enhance the activity of the expression control region, or replacing the sequence with a sequence having stronger activity. The expression control region may include, but is not particularly limited to, a promoter, an operator sequence, a sequence encoding a ribosome binding site, a sequence controlling the termination of transcription and translation, and the like. For example, the replacing may be replacing the original promoter with a strong promoter, but is not limited thereto.

Examples of the known stronger promoter may include CJ1 to CJ7 promoters (US 7662943 B2), lac promoter, trp promoter, trc promoter, tac promoter, lamda phage PR promoter, PL promoter, tet promoter, gapA promoter, SPL7 promoter, SPL13 (sm3) promoter (US 10584338 B2), O2 promoter (US 10273491 B2), tkt promoter, yccA promoter, and the like, but are not limited thereto.

The modifying of the nucleotide sequence encoding the initiation codon or 5'-UTR of the gene transcript encoding the polypeptide in item 3) above may be, for example, replacing the nucleotide sequence with a nucleotide sequence encoding another initiation codon having a higher expression rate of the polypeptide compared with the endogenous initiation codon, but is not limited thereto.

The modifying of an amino acid sequence or polynucleotide sequence in items 4) and 5) above may be inducing a modification on the sequence through deletion, insertion, non-conservative or conservative substitution, or a combination thereof in the amino acid sequence of the polypeptide or the polynucleotide sequence encoding the polypeptide to further enhance the activity of the polypeptide, or replacing the sequence with an amino acid sequence or polynucleotide sequence modified to have stronger activity or an amino acid sequence or polynucleotide sequence modified to enhance the activity thereof, but are not limited thereto. Specifically, the replacing may be performed by inserting the polynucleotide into the chromosome by homologous recombination, but is not limited thereto. The vector used herein may further include a selection maker for identifying the insertion of the chromosome. The selection marker is as described above.

The introducing of an exogenous polypeptide exhibiting the activity of the polypeptide in item 6) above may be introducing, into a microorganism, of an exogenous polynucleotide encoding a polypeptide exhibiting the same/similar activity to the polypeptide. The exogenous polynucleotide is not limited to the origin or sequence thereof as long as the exogenous polynucleotide exhibits the same/similar activity to the polynucleotide. The introduction may be performed by any known transformation method appropriately selected by a person skilled in the art, and the introduced polynucleotide is expressed in a host cell and thus the polypeptide is produced and the activity thereof can be enhanced.

As used herein, the term "introducing" refers to a method of delivering a polynucleotide encoding the acetohydroxy acid synthase small subunit variant or a vector including the same to a microorganism. Such introduction may be easily performed by a common method in the art. In general, examples of the method include a CaCl₂ precipitation method, a Hanahan method with improved efficiency using dimethyl sulfoxide (DMSO) as a reducing agent in the CaCl₂ precipitation method, an electroporation method, a calcium phosphate precipitation method, a protoplast fusion method, a stirring method using silicon carbide fibers, a transformation method using PEG, a dextran sulfate-, lipofectamine-, and dry/suppression-mediated transformation method, and the like. The method for transforming the vector is not limited to the above exemplified methods, and any transformation or transfection method commonly used in the art may be used without limitation. The delivered polynucleotide may be inserted into the chromosome of a microorganism and located therein or may be outside the chromosome, as long as the polynucleotide can be expressed in the host cell. Alternatively, the polynucleotide may be introduced in any form as long as it can be introduced into the host cell and expressed therein. For example, the polynucleotide may be introduced into a microorganism in the form of an expression cassette, which is a polynucleotide construct including all the essential elements required for its self-expression, but is not limited thereto. The expression cassette conventionally includes a promoter operably linked to the open reading frame (hereinafter, "ORF") of the gene, a transcription termination signal, a ribosome-binding domain, and a translation termination signal. The expression cassette may be in the form of a self-replicable expression vector. The polynucleotide may be introduced in the form as it is into a microorganism and operably linked to a sequence required for its expression in the microorganism, but is not particularly limited thereto.

The codon optimization of a polynucleotide encoding the polypeptide in item 7) may be the codon optimization of an endogenous polynucleotide so as to increase transcription or translation thereof in a microorganism, or the codon optimization of an exogenous polynucleotide so as to perform optimized transcription or translation in a microorganism.

The modifying or chemical modifying of an exposed site selected by analysis of a tertiary structure of the polypeptide in item 8) above may be, for example, modifying or chemical modifying an exposed site to be modified or chemically modified by comparing sequence information of a polypeptide to be analyzed with a database that stores sequence information of existing proteins to determine a template protein candidate according to similarity of the sequences and identifying the structure on the basis of the determined candidate.

Such enhancement of the activity of the polypeptide may mean that the activity, concentration, expression level of the corresponding polypeptide is increased relative to the activity or concentration of the polypeptide expressed in a wild type microbial strain or a microbial strain before modification, or that the amount of a product produced from the corresponding polypeptide is increased, but is not limited thereto.

In the microorganism of the present application, the modification of the polynucleotide as a whole or in part may be induced by (a) homologous recombination using a vector for chromosomal insertion into the microorganism or genome editing using an engineered nuclease (*e.g*., CRISPR-Cas9) and/or (b) treatment with light, such as UV rays and radioactive rays, and/or chemicals, without being limited thereto. A method of modifying the gene as a whole or in part may include a method by DNA recombinant technology. For example, a part of or the entire gene may be deleted by injecting a nucleotide sequence or vector containing a nucleotide sequence homologous to a target gene into the microorganism to cause homologous recombination. The injected nucleotide sequence or vector may contain a dominant selection marker, but is not limited thereto.

In the microorganism of the present application, the variant, polynucleotide, L-valine, and the like are as described in the other aspects.

In accordance with still another aspect of the present application, there is provided a method for producing L-valine, the method including culturing the microorganism in a medium.

Specifically, the method for producing L-valine of the present application may include culturing, in a medium, a *Corynebacterium glutamicum* strain including the variant of the present application, the polynucleotide of the present application, or the vector of the present application, but is not limited thereto.

As used herein the term "culturing" refers to growing the microorganism of the present application in appropriately adjusted environment conditions. The culturing of the present application may be performed according to appropriate media or culture conditions known in the art. Such culturing may be easily adjusted, according to the selected strain, by a person skilled in the art. Specifically, the culturing may be performed in a batch type, a continuous type, and/or a fed-batch type, but is not limited thereto.

As used herein, the term "medium" refers to a mixture containing, as main ingredients, nutrient materials required for culturing the microorganism of the present application, wherein the medium supplies nutrient materials, growth factors, and the like, including water that is essential for survival and growth. Specifically, the medium and other culture conditions for culturing the microorganism of the present application may be any medium that is used for common culturing microorganisms without particular limitation. However, the microorganism of the present application may be cultured in a common medium containing appropriate carbon sources, nitrogen sources, phosphorus sources, inorganic compounds, amino acids, and/or vitamins, under aerobic conditions, while the temperature, pH, and the like are adjusted.

For example, culture media for strains of the genus *Corynebacterium* may be found in the literature ("Manual of Methods for General Bacteriology" by the American Society for Bacteriology (Washington D.C., USA, 1981)].

In the present application, examples of the carbon sources may include: carbohydrates, such as glucose, saccharose, lactose, fructose, sucrose, and maltose; sugar alcohols, such as mannitol and sorbitol; organic acids, such as pyruvic acid, lactic acid, and citric acid; and amino acids, such as glutamic acid, methionine, and lysine. In addition, natural organic nutrient sources may be used, such as starch hydrolysates, molasses, blackstrap molasses, rice bran, cassava, bagasse, and corn steep liquor, and specifically, carbohydrates, such as glucose and sterile pretreated molasses (*i.e*., molasses converted to reduced sugars) may be used, and appropriate amounts of other carbon sources may be used without limitation. These carbon sources may be used alone or in combination of two or more, but is not limited thereto.

Examples of the nitrogen sources may include: inorganic nitrogen sources, such as ammonia, ammonium sulfate, ammonium chloride, ammonium acetate, ammonium phosphate, ammonium carbonate, and ammonium nitrate; amino acids, such as glutamic acid, methionine, and glutamine; and organic nitrogen sources, such as peptone, NZ-amine, meat extracts, yeast extracts, malt extracts, corn steep liquor, casein hydrolysates, fishes or decomposition products thereof, defatted soybean cake or decomposition products thereof. These nitrogen sources may be used alone or in combination of two or more thereof, but are not limited thereto.

Examples of the phosphorus sources may include potassium phosphate monobasic, potassium phosphate dibasic, and sodium-containing salts corresponding thereto. Examples of the inorganic compounds may include sodium chloride, calcium chloride, iron chloride, magnesium sulfate, iron sulfate, manganese sulfate, calcium carbonate, and the like, and besides, may include amino acids, vitamins, and/or suitable precursors. These constituent ingredients or precursors may be added to the medium in a batch or continuous manner. However, examples of the phosphorus sources are not limited thereto.

The pH of the medium may be adjusted by adding compounds, such as ammonium hydroxide, potassium hydroxide, ammonia, phosphoric acid, and sulfuric acid, to the medium in an appropriate manner during culturing of the microorganism of the present application. In addition, an anti-foaming agent, such as a fatty acid polyglycol ester, may be added to suppress foam formation during culturing. Additionally, oxygen or oxygen-containing gas may be injected into the medium to maintain the aerobic state of the medium, or nitrogen, hydrogen or carbon dioxide gas may be injected without the injection of gas to maintain the anaerobic or non-aerobic state of the medium, but is not limited thereto.

In the present application, the temperature for culturing may be maintained at 20°C to 45°C, and specifically 25°C to 40°C, and culturing may be performed for about 10 hours to 160 hours, but is not limited thereto.

L-Valine produced by culturing of the present application may be released into the medium or may remain in cells.

The method for producing L-valine of the present application may further include preparing the microorganism of the present application, preparing the medium for culturing the strain, or a combination of these steps (regardless of the order, in any order), for example, before the culturing step.

The method for producing L-valine of the present application may further include recovering L-valine from the medium resulting from the culturing (a medium where culturing has been performed) or the microorganism of the present application. The method of the present application may further include the recovering step after the culturing step.

The recovering may be collecting desired L-valine by using an appropriate method known in the art according to the method for culturing the microorganism of the present application, for example, a batch, continuous, or fed-batch type of culturing. Examples of the method may include centrifugation, filtration, treatment with a crystallized protein precipitating agent (salting-out), extraction, ultrasonic disruption, ultrafiltration, dialysis, various types of chromatography, such as molecular sieve chromatography (gel filtration), adsorption chromatography, ion exchange chromatography, and affinity chromatography, HPLC, and a combination of these methods, and desired L-valine can be recovered from the medium or microorganism by using an appropriate method known in the art.

The method for producing L-valine of the present application may further include a purification step. The purification may be performed by using an appropriate method known in the art. As an example, when the method for producing L-valine of the present application includes both the recovery step and the purification step, the recovery step and the purification step may be performed continuously or discontinuously regardless of the order, or may be performed simultaneously or integrated into one step, but is not limited thereto.

In the method of the present application, the variant, polynucleotide, vector, strain, and the like are as described in the other aspects.

Still another aspect of the present application is to provide a composition for producing L-valine, the composition containing a microorganism including the variant of the present application, a polynucleotide encoding the variant, a vector containing the polynucleotide, or the polynucleotide of the present application; a medium obtained by culturing the microorganism; or a combination of two or more thereof.

The composition of the present application may further contain any appropriate excipient that is commonly used in compositions for producing amino acids, and examples of the excipient may include a preserving agent, a wetting agent, a dispersing agent, a suspending agent, a buffer, a stabilizing agent, or an isotonic agent, but are not limited thereto.

In the composition of the present application, the variant, polynucleotide, vector, strain, medium, L-valine, and the like are as described in the other aspects.

In accordance with another aspect of the present application, there is provided use of the acetohydroxy acid synthase small subunit variant for producing L-valine, the variant having a substitution of the amino acid at position 44 in the amino acid sequence of SEQ ID NO: 1 with another amino acid.

In the use of the present application, the variant, L-valine, and the like are as described in the other aspects.

### [Mode for Carrying Out the Invention]

Hereinafter, the present application will be described in detail with reference to examples. However, these examples are merely preferable embodiments for illustrating the present application, and therefore are not intended to limit the scope of right of the present application. Meanwhile, the technical matters not described herein can be sufficiently understood and easily implemented by a person skilled in the art or similar technical fields of the present application.

### Example 1. Selection of mutant strain with increased valine producing ability through artificial mutation

### Example 1-1. Artificial mutagenesis through UV irradiation

In order to select a mutant strain with increased valine producing ability, *Corynebacterium glutamicum* KCCM11201P (Korean Patent Publication No. 10-1117022), a valine-producing strain, was plated on a nutrient medium containing agar and cultured at 30°C for 36 hours. Hundreds of colonies thus obtained were irradiated with UV at room temperature to induce random mutagenesis on the genome in the strain.

### Example 1-2. Evaluation of fermentation titers of mutated strains and selection of strain

In order to select mutant strains having increased L-valine producing ability compared with *Corynebacterium glutamicum* KCCM11201P used as a parent strain, a fermentation titer test was performed on randomly mutated strains. After each colony was subcultured in a nutrient medium, each strain was inoculated into a 250 mL corner-baffle flask containing 25 mL of a production medium and cultured with shaking at 200 rpm for 72 hours at 30°C. Thereafter, the concentration of L-valine was analyzed using HPLC, and the analyzed concentrations of L-valine were tabulated in Table 1.

### [Nutrient medium (pH 7.2)]

glucose 10 g, meat juice 5 g, polypeptone 10 g, sodium chloride 2.5 g, yeast extract 5 g, agar 20 g, and urea 2 g (based on 1 L of distilled water)

### [Production medium (pH 7.0)]

glucose 100 g, ammonium sulfate 40 g, soy protein 2.5 g, corn steep solids 5 g, urea 3 g, potassium phosphate dibasic 1 g, magnesium sulfate heptahydrate 0.5 g, biotin 100 µg, thiamine-HCl 1 mg, calcium pantothenate 2 mg, nicotine amide 3 mg, calcium carbonate 30 g (based on 1 L of distilled water)

**TABLE 1**

| | Strain name | L-Valine (g/L) |
|---|---|---|
| Control | KCCM11201P | 2.8 |
| Experimental groups | C1 | 3.1 |
| | C2 | 3.3 |
| | C3 | 2.5 |
| | C4 | 2.4 |
| | C5 | 2.3 |
| | C6 | 1.2 |
| | C7 | 2.9 |
| | C8 | 3.4 |
| | C9 | 3.2 |
| | C10 | 3.2 |
| | C11 | 1.9 |
| | C12 | 2.4 |
| | C13 | 3.8 |
| | C14 | 4.5 |
| | C15 | 1.8 |
| | C16 | 2.3 |
| | C17 | 2.7 |
| | C18 | 2.6 |
| | C19 | 2.6 |
| | C20 | 3.5 |
| | C21 | 2.5 |

On the basis of the results shown in Table 1, C14 strain, with the highest increase in the amount of valine production compared with KCCM1 1201P strain as a control, was selected.

### Example 2. Identification of mutation through gene sequencing

The main genes of the strain were sequenced and compared with those of KCCM11201P strain and wild-type *Corynebacterium glutamicum* ATCC14067 strain. The results identified that the KCCM11201P strain and the C14 strain with increased valine producing ability included nucleotide sequence mutations at specific positions of the open reading frame (ORF) region of the *ilvN* gene. Specifically, the KCCM11201P had a form in which the amino acid alanine at position 42 was substituted with valine, corresponding to the change of existing GCA into GTA by the introduction of one mutation into the nucleotide at position 125 from an initiation codon of the *ilvN* gene.

The C14 strain with the highest increase in the amount of valine production included A42V mutation included in the parent strain KCCM1 1201P, and had a form in which the amino acid threonine at position 44 was substituted with alanine, corresponding to the change of existing ACC into GCC by the introduction of one mutation into the nucleotide at position 130 from an initiation codon of the *ilvN* gene.

As a result of analyzing the mutation regions, the mutation regions were confirmed to affect the effector binding domain of valine bio-synthase, expecting the enhancement of the activity of the corresponding protein. In the following examples, an attempt was made to investigate the individual effects of the A42V and T44A mutations inserted at particular positions of ORF in the *ilvN* gene and the effect of the combined application on the ability of a microorganism of the genus *Corynebacterium* to produce valine, a branched-chain amino acid. In addition, an attempt was made to investigate the effects of a substitution with an amino acid other than alanine with respect to the mutation of threonine, the amino acid at position 44, on the ability of a microorganism of the genus *Corynebacterium* to produce the branched-chain amino acids valine, isoleucine, and leucine.

### Example 3. Preparation of KCCM11201P strains with introduction of ilvN mutations and identification of valine producing ability

### Example 3-1. Preparation of strain with introduction of ilvN mutation into Corynebacterium glutamicum KCCM11201P strain and evaluation of L-valine producing ability

In order to insert a ilvN(A42V+T44A) mutant set forth in SEQ ID NO: 6 into *Corynebacterium glutamicum* KCCM1 1201P, a vector containing a target mutation was constructed. Specifically, genomic DNA of the C14 strain was extracted using a G-spin Total DNA Extraction Mini Kit (Intron, Cat. No. 17045) according to the protocol provided in the kit, and PCR was performed using the genomic DNA as a template. The conditions for PCR were as follows: denaturation at 94°C for 5 minutes; 25 cycles of denaturation at 94°C for 30 seconds, annealing at 55°C for 30 seconds, and polymerization at 72°C for 150 seconds; and then polymerization at 72°C for 7 minutes. A PCR product (hereinafter referred to as "mutated fragment 1") of 1010 bp was obtained using SEQ ID NOS: 7 and 8.

The obtained mutated fragment 1 was ligated to a pDZ vector (Korean Patent Publication No. 10-0924065 and International Patent Publication No. 2008-033001) treated with the restriction enzyme Xbal (New England Biolabs, Beverly, MA) by using an Infusion Cloning Kit (Takara Bio Inc., Otsu, Japan), followed by transformation into *E. coli* DH5α. The prepared gene was transformed into *E*. *coli* DH5α, and then the transformed strains were selected in an LB medium containing kanamycin, and DNA was obtained therefrom by a DNA-spin plasmid DNA purification kit (iNtRON), thereby constructing pDZ-ilvN(A42V+T44A) vector containing the mutated fragment 1.

**TABLE 2**

| Primer | Nucleotide sequence |
|---|---|
| SEQ ID NO: 7 | cggggatcctctaga AGGACGGTACTCAAATACTAAACTTC |
| SEQ ID NO: 8 | cggggatcctctaga GACAACTACATTATTATTATACCACA |

Specifically, the pDZ-ilvN(A42V+T44A) vector was transformed into the *Corynebacterium glutamicum* KCCM11201P by homologous recombination on the chromosome (van der Rest et al., Appl. Microbiol Biotechnol 52:541-545, 1999). Strains with the vector inserted into the chromosome by homologous sequence recombination were selected in a medium containing kanamycin (25 mg/L). Thereafter, PCR using SEQ ID NOS: 7 and 8 was performed on the *Corynebacterium glutamicum* transformants subjected to secondary recombination to identify a strain in which alanine was substituted with valine at position 42 and threonine was substituted with alanine at position 44 in the amino acid sequence of SEQ ID NO: 1 within ORF of the *ilvN* gene on the chromosome, respectively. The recombinant strain was named *Corynebacterium glutamicum* KCCM11201P::*ilvN*(A42V+T44A).

To compare L-valine producing abilities of the valine-producing strains *Corynebacterium glutamicum* KCCM11201P and KCCM11201P::*ilvN*(A42V+T44A), flask evaluation was performed. Each strain was subcultured in a nutrient medium, inoculated into a 250 mL corner-baffle flask containing 25 mL of a production medium, and cultured with shaking at 200 rpm for 72 hours at 30°C. Thereafter, the concentration of L-valine was analyzed using HPLC, and the analyzed concentrations of L-valine were tabulated in Table 3.

### [Nutrient medium (pH 7.2)]

glucose 10 g, meat juice 5 g, polypeptone 10 g, sodium chloride 2.5 g, yeast extract 5 g, agar 20 g, and urea 2 g (based on 1 L of distilled water)

### [Production medium (pH 7.0)]

glucose 100 g, ammonium sulfate 40 g, soy protein 2.5 g, corn steep solids 5 g, urea 3 g, potassium phosphate dibasic 1 g, magnesium sulfate heptahydrate 0.5 g, biotin 100 µg, thiamine-HCl 1 mg, calcium pantothenate 2 mg, nicotine amide 3 mg, calcium carbonate 30 g (based on 1 L of distilled water)

**TABLE 3**

| L-Valine producing abilities of KCCM11201P and KCCM11201P::*ilvN* (A42V+T44A) | | | | |
|---|---|---|---|---|
| Strain | L-Valine (g/L) | | | |
| | Batch 1 | Batch 2 | Batch 3 | Mean |
| KCCM11201P | 2.7 | 2.7 | 2.8 | 2.7 |
| KCCM1201P::*ilvN*(A42V+T44A) | 3.3 | 3.2 | 3.1 | 3.2 |

The results identified that the L-valine producing ability of the KCCM11201P::*ilvN*(A42V+T44A) strain showed an 18.5% increase compared with KCCM11201P.

### Example 3-2. Preparation of strain with introduction of ilvN mutation into Corynebacterium glutamicum KCCM11201P strain and evaluation of L-valine producing ability

In order to insert a ilvN(T44A) mutant set forth in SEQ ID NO: 3 into *Corynebacterium glutamicum* KCCM1 1201P, a vector containing a target mutation was constructed. Specifically, genomic DNA of the ATCC14067 strain, wild-type *Corynebacterium glutamicum*, was extracted using a G-spin Total DNA Extraction Mini Kit (Intron, Cat. No 17045) according to the protocol provided in the kit, and PCR was performed using the genomic DNA as a template. The conditions for PCR were as follows: denaturation at 94°C for 5 minutes; 25 cycles of denaturation at 94°C for 30 seconds, annealing at 55°C for 30 seconds, and polymerization at 72°C for 150 seconds; and then polymerization at 72°C for 7 minutes. A PCR product (hereinafter referred to as "mutated fragment 2") of 515 bp was obtained using SEQ ID NOS: 9 and 10, and a PCR product (hereinafter referred to as "mutated fragment 3") of 518 bp was obtained using SEQ ID NOS: 11 and 12.

The obtained mutated fragments 2 and 3 were ligated to a pDZ vector (Korean Patent Publication No. 10-0924065 and International Patent Publication No. 2008-033001) treated with the restriction enzyme Xbal (New England Biolabs, Beverly, MA) by using an Infusion Cloning Kit (Takara Bio Inc., Otsu, Japan), followed by transformation into *E. coli* DH5α. The prepared gene was transformed into *E. coli* DH5α, and then the transformed strains were selected in an LB medium containing kanamycin, and DNA was obtained therefrom by a DNA-spin plasmid DNA purification kit (iNtRON), thereby constructing pDZ-ilvN(T44A) vector containing the mutated fragments 2 and 3.

**TABLE 4**

| Primer | Nucleotide sequence |
|---|---|
| SEQ ID NO: 9 | cggggatcctctaga AGGACGGTACTCAAATACTAAACTTC |
| SEQ ID NO: 10 | GGCCTTTGCAGACACGAGGGACACGAGG |
| SEQ ID NO: 11 | TGTCCCTCGTGTCTGCAAAGGCCGAAACACTCGGC |
| SEQ ID NO: 12 | cggggatcctctaga GACAACTACATTATTATTATACCACA |

Specifically, the pDZ-ilvN(T44A) vector was transformed into the *Corynebacterium glutamicum* KCCM11201P by homologous recombination on the chromosome (van der Rest et al., Appl. Microbiol Biotechnol 52:541-545, 1999). The homologous recombination resulted in the restoration from A42V mutation and the introduction of T44A mutation. Strains with the vector inserted into the chromosome by homologous sequence recombination were selected in a medium containing kanamycin (25 mg/L). Thereafter, PCR using SEQ ID NOS: 9 and 12 was performed on the *Corynebacterium glutamicum* transformants subjected to secondary recombination to identify a strain in which threonine was substituted with alanine at position 44 in the amino acid sequence of SEQ ID NO: 1 within ORF of the *ilvN* gene on the chromosome. The recombinant strain was named *Corynebacterium glutamicum* KCCM11201P::*ilvN*(T44A).

To compare L-valine producing abilities of the prepared strains, the strains were cultured and the concentration of L-valine was analyzed by the same method as in Example 3-1, and the analyzed concentrations of L-valine were tabulated in Table 5 below.

**TABLE 5**

| L-Valine producing abilities of KCCM11201P, KCCM11201P::*ilvN*(T44A), and KCCM11201P::*ilvN*(A42V+T44A) | | | | |
|---|---|---|---|---|
| Strain | L-Valine (g/L) | | | |
| | Batch 1 | Batch 2 | Batch 3 | Mean |
| KCCM11201P | 2.7 | 2.7 | 2.8 | 2.7 |
| KCCM11201P::*ilvN*(T44A) | 2.9 | 3.0 | 2.9 | 2.9 |
| KCCM11201P::*ilvN*(A42V+T44A) | 3.2 | 3.3 | 3.1 | 3.2 |

The results identified that the L-valine producing abilities of the KCCM11201P::*ilvN*(T44A) and KCCM11201P::*ilvN*(A42V+T44A) strains showed 7.4% and 18.5% increases compared with KCCM11201P, respectively.

### Example 3-3. Preparation of strain with introduction of ilvN mutation into Corynebacterium glutamicum CJ7V strain and evaluation of L-valine producing ability

To identify whether the mutation had an effect of increasing L-valine producing ability even in other *Corynebacterium glutamicum* strains producing L-valine, a strain with improved L-valine producing ability was prepared by the introduction of one type of mutation (ilvN(A42V); Biotechnology and Bioprocess Engineering, June 2014, Volume 19, Issue 3, pp. 456-467) into the wild-type *Corynebacterium glutamicum* ATCC14067.

Specifically, genomic DNA of the ATCC14067 strain, wild-type *Corynebacterium glutamicum*, was extracted using a G-spin Total DNA Extraction Mini Kit (Intron, Cat. No. 17045) according to the protocol provided in the kit. PCR was performed using the genomic DNA as a template. To construct a vector for introducing the A42V mutation into the *ilvN* gene, gene fragments (A and B) were obtained using a primer pair of SEQ ID NOS: 13 and 14 and a primer pair of SEQ ID NOS: 15 and 16, respectively. The conditions for PCR were as follows: denaturation at 94°C for 5 minutes; 25 cycles of denaturation at 94°C for 30 seconds, annealing at 55°C for 30 seconds, and polymerization at 72°C for 60 seconds; and then polymerization at 72°C for 7 minutes.

As a result, polynucleotides of 528 bp and 509 bp could be obtained for fragments A and B, respectively. Overlapping PCR using the two fragments as a template along with SEQ ID NOS: 13 and 16 was performed to obtain a PCR product of 1010 bp (hereinafter referred to as "mutated fragment 4").

The obtained mutated fragment 4 was treated with the restriction enzyme *Xbal* (New England Biolabs, Beverly, MA), and then ligated to pDZ vector treated with the same restriction enzyme by T4 ligase (New England Biolabs, Beverly, MA). The prepared gene was transformed into *E. coli* DH5α, and then the transformed strains were selected in an LB medium containing kanamycin, and DNA was obtained therefrom by a DNA-spin plasmid DNA purification kit (iNtRON). The vector having a purpose of the introduction of the A42V into the *ilvN* gene was named pDZ-ilvN(A42V).

**TABLE 6**

| Primer | Nucleotide sequence |
|---|---|
| SEQ ID NO: 13 | cggggatcctctagaAGGACGGTACTCAAATACTAAACTTC |
| SEQ ID NO: 14 | TGCCGAGTGTTTCGGTCTTTACAGACACGAGGGAC ACG |
| SEQ ID NO: 15 | TGTCTGTAAAGACCGAAACACTCGGCATCAA |
| SEQ ID NO: 16 | cggggatcctctagaGACAACTACATTATTATTATACCACA |

Thereafter, the pDZ-ilvN(A42V) vector was transformed into the wild-type *Corynebacterium glutamicum* ATCC14067 by homologous recombination on the chromosome (van der Rest et al., Appl. Microbiol Biotechnol 52:541-545, 1999). Strains with the vector inserted into the chromosome by homologous sequence recombination were selected in a medium containing kanamycin (25 mg/L). Thereafter, PCR using SEQ ID NOS: 13 and 16 was performed on the *Corynebacterium glutamicum* transformants subjected to secondary recombination to amplify the gene fragment and then a mutation-inserted strain was identified through gene sequencing. The recombinant strain was named *Corynebacterium glutamicum* CJ7V.

Last, the *Corynebacterium glutamicum* CJ7V was transformed with the respective vectors by the same methods as in Example 3-1 and Example 3-2, respectively, and the strain transformants were named *Corynebacterium glutamicum* CJ7V::*ilvN*(T44A) and CJ7V::*ilvN* (A42V+T44A), respectively. To compare L-valine producing abilities of the prepared strains, the strains were cultured and the concentration of L-valine was analyzed by the same method as in Example 3-1, and the analyzed concentrations of L-valine were tabulated in Table 7 below.

**TABLE 7**

| L-Valine producing abilities of CJ7V, CJ7V::*ilvN*(T44A), and CJ7V::*ilvN* (A42V+T44A) | | | | |
|---|---|---|---|---|
| Strain | L-Valine (g/L) | | | |
| | Batch 1 | Batch 2 | Batch 3 | Mean |
| *CJ7V* | 3.5 | 3.5 | 3.6 | 3.5 |
| CJ7V::*ilvN*(T44A) | 3.9 | 3.8 | 3.8 | 3.8 |
| CJ7V::*ilvN* (A42V+T44A) | 4.1 | 4.0 | 4.0 | 4.0 |

The results identified that the L-valine producing abilities of the CJ7V::*ilvN*(T44A) and CJ7V::*ilvN* (A42V+T44A) strains showed 8.5% and 12.3% increases compared with CJ7V, respectively.

As set forth above, a person skilled in the art to which the present application pertains will be able to understand that the present application may be embodied in other specific forms without departing from the technical spirit or essential characteristics thereof. Therefore, the embodiments described above should be construed as being exemplified and not limiting the present application. The scope of the present application should be understood that all changes or modifications derived from the definitions and scopes of the claims and their equivalents fall within the scope of the application.

### SEQUENCE LISTING

| SEQ ID NO | Name | Sequence |
|---|---|---|
| 1 | Acetohydroxy acid synthase small subunit amino acid sequence (WT) | MANSDVTRHILSVLVQDVDGIISRVSGMFTRRAFNLV SLVSAKTETLGINRITVVVDADELNIEQITKQLNKLIPVL KVVRLDEETTIARAIMLVKVSADSTNRPQIVDAANIFRA RVVDVAPDSVVIESTGTPGKLRALLDVMEPFGIRELIQ SGQIALNRGPKTMAPAKI |
| 2 | Acetohydroxy acid synthase small subunit polynucleotide sequence (WT) | ATGGCTAATTCTGACGTCACCCGCCACATCCTGTC CGTACTCGTTCAGGACGTAGACGGAATCATTTCCC GCGTATCAGGTATGTTCACCCGACGCGCATTCAAC CTCGTGTCCCTCGTGTCTGCAAAGACCGAAACACT CGGCATCAACCGCATCACGGTTGTTGTCGACGCCG ACGAGCTCAACATTGAGCAGATCACCAAGCAGCTC AACAAGCTGATCCCCGTGCTCAAAGTCGTGCGACT TGATGAAGAGACCACCATCGCCCGCGCAATCATGC TGGTTAAGGTCTCTGCGGATAGCACCAACCGTCCG CAGATCGTCGACGCCGCGAACATCTTCCGCGCCC GAGTCGTCGACGTGGCTCCAGACTCTGTGGTTATT GAATCCACAGGCACCCCAGGCAAGCTCCGCGCAC TGCTTGATGTGATGGAACCATTCGGAATCCGCGAA CTGATCCAATCCGGACAGATTGCACTCAACCGCGG TCCGAAGACCATGGCTCCGGCCAAGATCTAA |
| 3 | Acetohydroxy acid synthase small subunit variant (T44A) amino acid sequence | MANSDVTRHILSVLVQDVDGIISRVSGMFTRRAFNLV SLVSAKAETLGINRITVVVDADELNIEQITKQLNKLIPVL KVVRLDEETTIARAIMLVKVSADSTNRPQIVDAANIFRA RVVDVAPDSVVIESTGTPGKLRALLDVMEPFGIRELIQ SGQIALNRGPKTMAPAKI |
| 4 | Acetohydroxy acid synthase small subunit variant (T44A) polynucleotide sequence | ATGGCTAATTCTGACGTCACCCGCCACATCCTGTC CGTACTCGTTCAGGACGTAGACGGAATCATTTCCC GCGTATCAGGTATGTTCACCCGACGCGCATTCAAC CTCGTGTCCCTCGTGTCTGCAAAGGCCGAAACACT CGGCATCAACCGCATCACGGTTGTTGTCGACGCCG ACGAGCTCAACATTGAGCAGATCACCAAGCAGCTC |
| | | AACAAGCTGATCCCCGTGCTCAAAGTCGTGCGACT TGATGAAGAGACCACCATCGCCCGCGCAATCATGC TGGTTAAGGTCTCTGCGGATAGCACCAACCGTCCG CAGATCGTCGACGCCGCGAACATCTTCCGCGCCC GAGTCGTCGACGTGGCTCCAGACTCTGTGGTTATT GAATCCACAGGCACCCCAGGCAAGCTCCGCGCAC TGCTTGATGTGATGGAACCATTCGGAATCCGCGAA CTGATCCAATCCGGACAGATTGCACTCAACCGCGG TCCGAAGACCATGGCTCCGGCCAAGATCTAA |
| 5 | Acetohydroxy acid synthase small subunit variant (T44A+A42V) amino acid sequence | MANSDVTRHILSVLVQDVDGIISRVSGMFTRRAFNLV SLVSVKAETLGINRITVVVDADELNIEQITKQLNKLIPVL KVVRLDEETTIARAIMLVKVSADSTNRPQIVDAANIFRA RVVDVAPDSVVIESTGTPGKLRALLDVMEPFGIRELIQ SGQIALNRGPKTMAPAKI |
| 6 | Acetohydroxy acid synthase small subunit variant (T44A+A42V) polynucleotide sequence | ATGGCTAATTCTGACGTCACCCGCCACATCCTGTC CGTACTCGTTCAGGACGTAGACGGAATCATTTCCC GCGTATCAGGTATGTTCACCCGACGCGCATTCAAC CTCGTGTCCCTCGTGTCTGTAAAGGCCGAAACACT CGGCATCAACCGCATCACGGTTGTTGTCGACGCCG ACGAGCTCAACATTGAGCAGATCACCAAGCAGCTC AACAAGCTGATCCCCGTGCTCAAAGTCGTGCGACT TGATGAAGAGACCACCATCGCCCGCGCAATCATGC TGGTTAAGGTCTCTGCGGATAGCACCAACCGTCCG CAGATCGTCGACGCCGCGAACATCTTCCGCGCCC GAGTCGTCGACGTGGCTCCAGACTCTGTGGTTATT GAATCCACAGGCACCCCAGGCAAGCTCCGCGCAC TGCTTGATGTGATGGAACCATTCGGAATCCGCGAA CTGATCCAATCCGGACAGATTGCACTCAACCGCGG TCCGAAGACCATGGCTCCGGCCAAGATCTAA |
| 7 | primer | cggggatcctctaga AGGACGGTACTCAAATACTAAACTTC |
| 8 | primer | cggggatcctctaga GACAACTACATTATTATTATACCACA |
| 9 | primer | cggggatcctctaga |
| | | AGGACGGTACTCAAATACTAAACTTC |
| 10 | primer | GGCCTTTGCAGACACGAGGGACACGAGG |
| 11 | primer | TGTCCCTCGTGTCTGCAAAGGCCGAAACACTCGGC |
| 12 | primer | cggggatcctctaga GACAACTACATTATTATTATACCACA |
| 13 | primer | cggggatcctctagaAGGACGGTACTCAAATACTAAACTT C |
| 14 | primer | TGCCGAGTGTTTCGGTCTTTACAGACACGAGGGAC ACG |
| 15 | primer | TGTCTGTAAAGACCGAAACACTCGGCATCAA |
| 16 | primer | cggggatcctctagaGACAACTACATTATTATTATACCACA |

## Claims

1. An acetohydroxy acid synthase small subunit (ilvN) variant in which the amino acid corresponding to position 44 in the amino acid sequence of SEQ ID NO: 1 is substituted with another amino acid.

2. The variant of claim 1, wherein the amino acid corresponding to position 42 in the amino acid sequence of SEQ ID NO: 1 is further substituted with another amino acid.

3. The variant of claim 1, wherein the amino acid corresponding to position 44 in the amino acid sequence of SEQ ID NO: 1 is substituted with alanine.

4. The variant of claim 2, wherein the amino acids corresponding to positions 44 and 42 in the amino acid sequence of SEQ ID NO: 1 are substituted with alanine and valine, respectively.

5. The variant of claim 1, wherein the variant consists of the amino acid sequence of SEQ ID NO: 3.

6. The variant of claim 2, wherein the variant consists of the amino acid sequence of SEQ ID NO: 5.

7. A polynucleotide encoding the variant of any one of claims 1 to 6.

8. An expression vector comprising the polynucleotide of claim 7.

9. A microorganism comprising the variant of any one of claims 1 to 6 or a polynucleotide encoding the variant.

10. The microorganism of claim 9, wherein the microorganism has an increased L-valine producing ability compared with a microorganism comprising the polypeptide of SEQ ID NO: 1 or a polynucleotide encoding the polypeptide.

11. The microorganism of claim 9, wherein the microorganism is a microorganism of the genus *Corynebacterium.*

12. The microorganism of claim 11, wherein the microorganisms of the genus *Corynebacterium* is *Corynebacterium glutamicum.*

13. A method for producing L-valine, the method comprising culturing the microorganism of claim 9 in a medium.

14. The method of claim 13, further comprising recovering a target substance from the medium.
